(19) Europäisches Patentamt
European Patent Office
Office européen des brevets

(11) Numéro de publication : **0 330 527 B1**

(12) **FASCICULE DE BREVET EUROPEEN**

(45) Date de publication du fascicule du brevet : **23.09.92 Bulletin 92/39**

(51) Int. Cl.$^5$ : **C07C 323/00**

(21) Numéro de dépôt : **89400157.7**

(22) Date de dépôt : **19.01.89**

(54) **Procédé de préparation de solutions aqueuses de l'acide 2-hydroxy-4-méthylthio-butyrique.**

(30) Priorité : **22.02.88 ES 8800496**

(43) Date de publication de la demande : **30.08.89 Bulletin 89/35**

(45) Mention de la délivrance du brevet : **23.09.92 Bulletin 92/39**

(84) Etats contractants désignés : **AT BE CH DE ES FR GB GR IT LI LU NL SE**

(56) Documents cités :
**EP-A- 0 143 100**
**EB-A- 771 153**
**US-A- 3 175 000**

(73) Titulaire : **SOCIEDAD DE DESARROLLO TECHNICO INDUSTRIAL**
**Poligono Villalonquejar C/.Merindad de Castilla la Vieja s/n**
**E-09080 Burgos (ES)**

(72) Inventeur : **Azagra Hernandez, Javier**
**Julio Saez de la Hoya, 6**
**Burgos (ES)**
Inventeur : **Ruiz Moreno, Luis**
**Virgen del Monzano, 6**
**Burgos (ES)**

(74) Mandataire : **Le Pennec, Magali**
**RHONE-POULENC RORER SA, Direction des Brevets, 20 Avenue Raymond Aron**
**F-92165 Antony Cédex (FR)**

**EP 0 330 527 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

### A. OBJET DE L'INVENTION

La présente invention concerne un procédé amélioré pour préparer des solutions aqueuses de l'acide DL-2-hydroxy-4-méthylthio-butyrique, ces solutions ayant moins de couleur et d'odeur, moins de viscosité et une bonne stabilité thermique quand on les compare au même produit préparé par d'autres procédés connus.

### B. ANTECEDENTS DE L'INVENTION

L'acide 2-hydroxy-4-méthylthio-butyrique (ci-après appelé AHMTB) possède la formule :

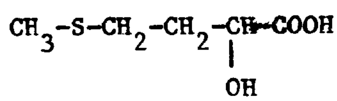

C'est un produit connu analogue à l'acide aminé essentiel DL-méthionine qui trouve son utilité principale comme supplément alimentaire pour l'alimentation des animaux. A la différence de l'acide aminé, il n'est pas utilisé directement par l'organisme dans la synthèse protéique, car il doit être transformé anaboliquement en acide aminé pour pouvoir être utilisé comme tel. Le AHMTB ne s'emploie pas sous la forme pure, mais sous diverses formes, à savoir :

1) concentrés qui contiennent un mélange des sels de calcium et d'ammonium dudit AHMTB, d'eau et de sulfate de calcium, comme c'est le cas dans les brevets US 2 745 745 et 2 938 053 et les brevets GB 722 024 et 915 193 ;

2) solutions aqueuses acides avec une concentration de AHMTB de 85-90 % en poids, comme cela est décrit dans les brevets US 4 353 924 et 3 773 927 ; et

3) le sel de calcium de AHMTB obtenu par le procédé du brevet US 3 175 000 du 23 Mars 1965.

### C. ETAT DE LA TECHNIQUE ANTERIEURE

Le procédé général d'obtention du AHMTB à partir de l'aldéhyde 3-méthylthio- propionique que l'on peut résumer par le schéma suivant :

(1) aldéhyde 3-méthylthio-propionique

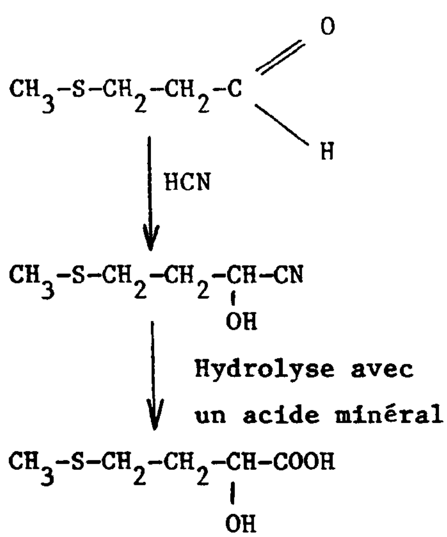


(2) 2-hydroxy-4-méthylthio-butyronitrile (appelé par abréviation HMTBN)

(3) acide 2-hydroxy-4-méthylthio-butyrique (AHMTB)

est amplement décrit dans la bibliographie des brevets, concrètement dans les brevets US 2 745 745 et 2 938 053 dans lesquels l'étape (2) → (3) du schéma précédent implique le passage par l'amide intermédiaire qui, moyennant une hydrolyse additionnelle, conduit au AHMTB. Cependant, en fonction des conditions d'hydrolyse employées, on obtient l'acide libre ou l'amide intermédiaire. Cette réaction d'hydrolyse du nitrile apparaît également décrite dans les brevets US 4 353 924 et 3 175 000 qui emploient spécifiquement l'acide sulfurique et dans le brevet US 3 773 927 qui emploie l'acide chlorhydrique. Lesdits brevets de la technique antérieure décrivent aussi les conditions opératoires du procédé, telles que la température, la durée et les rapports molaires de l'acide minéral au nitrile. Dans ces brevets de la technique antérieure, une fois obtenu le AHMTB par la réaction d'hydrolyse, on l'isole sous la forme indiquée dans le paragraphe (B) (sels ou solution aqueuse) au moyen d'opérations auxquelles on se réfèrera plus avant en comparant lesdits procédés de la technique antérieure avec le procédé de la présente invention.

Finalement, les demandes de brevets européens publiées sous les n° 0 142 488 et 0 143 100, toutes deux déposées le 13 Novembre 1984, se rapportent spécifiquement à l'obtention de formes liquides du AHMTB par des procédés d'extraction avec des solvants dudit AHMTB, du mélange de réaction d'hydrolyse du HMTBN réalisée dans le premier cas avec de l'acide sulfurique, et dans le deuxième cas avec un acide minéral. Les deux demandes de brevets européens décrivent avec tous les détails les conditions permettant de réaliser l'extraction avec solvant, ainsi que la nature exacte des solvants employés.

Cependant, les procédés de la technique antérieure repris dans les brevets et les demandes citées pâtissent de divers inconvénients dérivés de la manière spécifique de réaliser l'isolement du produit final AHMTB. Ces inconvénients peuvent se résumer comme suit :

a) Présence d'oligomères du AHMTB avec la concomitante mauvaise odeur, la couleur et la pauvreté en acide libre.

b) Viscosité excessive.

c) Présence de fines particules de sulfate de calcium et/ou de sels inorganiques solubles qui rendent impur le produit.

d) Coût élevé du produit final en raison de l'emploi de moyens tels que l'extraction avec des solvants.

e) Bas rendements de récupération du AHMTB.

Lesdits inconvénients sont surmontés par le procédé de l'invention qui, à partir du mélange d'hydrolyse du HMTBN par l'acide sulfurique, en une seule étape, permet d'arriver à une solution aqueuse de AHMTB, dépourvue des désavantages antérieurs grâce à l'emploi d'un nouveau processus d'isolement du AHMTB sous forme de solution aqueuse.

## D. DESCRIPTION DU PROCEDE OBJET DE L'INVENTION

Le procédé de synthèse du 2-hydroxy-4-méthylthio-butyronitrile (HMTBN), décrit par la suite, est une variante de celui exposé dans le brevet espagnol 393 625 pour la synthèse de l'acide aminé DL-méthionine, la synthèse du nitrile étant semblable dans les deux procédés sans autre différence que celle d'utiliser une solution aqueuse ammoniacale comme moyen de réaction pour obtenir le nitrile correspondant à l'acide aminé, et de l'eau exempte d'ammoniac pour le cas du HMTBN.

On peut dire la même chose de l'étape succédant à la synthèse, à savoir l'acidification du HMTBN. Dans les deux cas, le produit obtenu dans l'étape de synthèse est mis en contact avec de l'acide sulfurique concentré, de préférence à 98 %, dans une boucle d'acidification ou sont mélangés les deux produits. Afin d'éviter des échauffements locaux conduisant à la destruction du nitrile et à la formation de réactions secondaires entraînant une augmentation de coloration, l'acidification est réalisée en continu avec une grande recirculation, en ajoutant l'acide sulfurique concentré sur la solution acidifiée du nitrile, cette solution ayant de préférence une concentration de 20-50 % en poids d'acide.

Comme il est nécessaire d'éliminer la chaleur de dilution de l'acide sulfurique, la boucle d'acidification est pourvue d'un ou de plusieurs échangeurs de chaleur de façon que la température de réaction ne dépasse pas une valeur de 50°C.

Le rapport molaire acide sulfurique/HMTBN doit être compris entre 0,5-2, de préférence 0,8-1,5. La proportion d'eau doit être ajustée de telle manière que la solution acidifiée soit constituée par une seule phase et soit capable de maintenir en solution le sulfate d'ammonium qui va se former durant la réaction subséquente d'hydrolyse et la neutralisation postérieure avec de l'hydroxyde d'ammonium.

Après un temps de contact de 30-60 minutes, la solution est chauffée à une température comprise entre 60 et 140°C, de préférence 90°C, pour terminer la réaction d'hydrolyse du nitrile HMTBN en une seule étape, sans nécessité d'employer des conditions différentes pour obtenir l'amide intermédiaire, et de modifier ensuite lesdites conditions pour obtenir le AHMTB. La durée de réaction varie entre 5 minutes et 6 heures en fonction de la température choisie. Ainsi, par exemple, en employant 90°C et le rapport molaire susmentionné, on arrive à une transformation quantitative du HMTBN en AHMTB après environ 2 heures.

Il est extrêmement important de régulariser la température et la durée de réaction à l'intérieur des intervalles précités, vu que la couleur et la teneur en oligomères dépendent fondamentalement desdites valeurs en ce sens que des températures et des durées plus grandes que celles signalées conduisent à un produit avec plus de couleur et avec des valeurs plus élevées de la viscosité, ce qui est une donnée évidente d'une plus grande teneur en oligomères.

Pendant le déroulement de l'hydrolyse, il convient d'appliquer au réacteur un léger vide (entre environ 20 et 200 mm) de façon à éliminer le petit excès de HCN utilisé dans la synthèse du HMTBN, ainsi que les impuretés volatiles qui pourraient s'être formées dans la réaction et auxquelles on attribue la mauvaise odeur du produit final.

La réaction d'hydrolyse terminée, c'est alors que commence reellement l'isolement du AHMTB qui constitue l'aspect essentiel de la présente invention. Le mélange réactionnel d'hydrolyse est refroidi à 60-70°C, et son excès d'acide est neutralisé avec une solution d'ammoniac de 20-35 % en poids, ladite solution pouvant éventuellement être formée in situ par barbotage de $NH_3$ gazeux. Ladite neutralisation peut nécessiter un refroidissement pour ne pas dépasser la température de 60-70°C.

La masse neutralisée obtenue comprend deux phases facilement décantables, car elles présentent des densités nettement différentes. L'étape suivante du procédé est donc de séparer par décantation la phase organique qui contient 93-95 % du AHMTB formé, de la phase aqueuse qui renferme le reste. Le sulfate d'ammonium qui s'est formé durant la réaction d'hydrolyse et la neutralisation de l'acide utilisé en excès, se répartit aussi entre les deux phases, la phase aqueuse étant la plus riche avec 70-75 % en poids.

C'est une partie essentielle de la présente invention que de séparer les deux phases par décantation, centrifugation ou par un autre procédé standard de séparation liquide/liquide qui n'implique pas d'extraction avec des solvants, ce qui facilite extraordinairement les processus subséquents de purification et de récupération totale du AHMTB, d'une part, et du sulfate d' ammonium, d'autre part, avec un rendement et une efficacité élevés.

Des procédés séparateurs du type extraction avec un solvant non miscible à l'eau ne sont pas nécessaires ; ils n'améliorent pas substantiellement la qualité ni le rendement, mais augmentent le prix du processus et compliquent l'installation.

A partir de la phase aqueuse on précipite le sulfate d' ammonium par évaporation de l'eau à la pression atmosphérique ou sous pression réduite. Le solide résultant est séparé par n'importe quel procédé standard de séparation solide-liquide tel que la filtration et/ou la centrifugation, et le liquide ainsi obtenu, contenant la partie du sulfate d'ammonium qui n'a pas précipité et le AHMTB, est recyclé au récipient de neutralisation. Avec ce procédé on obtient du sulfate d'ammonium qui, une fois séché, possède une grande pureté et est pratiquement exempt de AHMTB ; ce dernier est récupéré dans sa totalité par recyclage en tête du processus de séparation, tout en restant incorporé à la phase organique.

La phase organique qui provient du décanteur et contient le AHMTB des deux flux (neutralisation et recyclage) est conduite à un évaporateur, travaillant sous vide, où l'eau est évaporée jusqu'à s'abaisser à 1 %, de préférence à 0,5 %, afin de précipiter la totalité du sulfate d'ammonium qu'elle contenait en solution. La suspension obtenue est parfaitement transportable, car elle contient en suspension seulement 25-30 % du sulfate d'ammonium produit au total dans les étapes de synthèse et neutralisation, et elle peut être séparée par filtration et/ou centrifugation avec une grande facilité. Le AHMTB obtenu par ce procédé est d'une grande pureté et sa teneur en sulfate d'ammonium est inférieure à 1 %.

Le tourteau de sulfate d'ammonium provenant du système séparateur susmentionné contient des quantités de AHMTB qui varient entre 15-30 % en poids. Les deux produits sont récupérés en les recyclant au récipient de neutralisation par dissolution préalable avec de l'eau et, de la même manière que pour le recyclage antérieur, le sulfate d'ammonium dissous est de préférence incorporé à la phase aqueuse et le AHMTB à la phase organique, ce qui fait que la récupération des deux produits est quantitative.

Le AHMTB obtenu par ce procédé doit être postérieurement dilué avec de l'eau et éventuellement stabilisé avec une petite quantité d'acide sulfurique, pour donner une solution qui contienne 65-95 % de AHMTB et entre 0,1 et 0,5 % d'acide sulfurique libre. La solution ainsi obtenue est en équilibre avec ses esters polymères ou oligomères et est stable dans le temps. Dans le produit final la relation entre le AHMTB monomère et la somme des oligomères est supérieure à 3, sa coloration est inférieure à 2000 degrés APHA et sa teneur en sulfate d'ammonium inférieure à 1 % en poids.

A titre purement illustratif, le procédé est maintenant décrit en faisant référence à l'unique figure des dessins représentant un diagramme de flux du procédé amélioré de l'invention. Le HMTBN représenté par (A) est introduit dans le récipient d'acidification (1), où il est mélangé avec de l'acide sulfurique concentré représenté par (B). La disposition de la boucle d'acidification formée par le récipient (1), les tuyauteries (C) et (D) et l'échangeur de chaleur (E) permet d'obtenir une concentration d'acide sulfurique de 20-50 % en poids dans le récipient (1). Par la tuyauterie (F) le mélange réactionnel passe au réacteur d'hydrolyse (2) dans lequel est réalisée la réaction dans les conditions de température et de temps spécifiées auparavant. Ledit réacteur d'hydrolyse est pourvu d'une communication avec l'atmosphère (G) pour ventilation, et d'une tuyauterie de sortie (H) qui conduit à un récipient de neutralisation et de mélange (3) dans lequel on introduit par (I) la solution d'hydroxyde d'ammonium (éventuellement on pourrait introduire de l'ammoniac gazeux pour former in situ la solution d'hydroxyde d'ammonium). A partir du récipient (3), la solution neutralisée passe par la tuyauterie (J) au décanteur

de phases (4), dans lequel se forment la phase organique (Ph O) et la phase aqueuse (Ph A).

La phase organique (Ph O) est amenée par la tuyauterie (K) à l'évaporateur sous vide (7), muni de son entrée de vapeur (EV) pour chauffage, de sa sortie pour le vide (SV) et de sa tuyauterie (N) pour effluent aqueux. Par la tuyauterie (M) sort une suspension transportable, constituée par du sulfate d'ammonium et du AHMTB ; cette suspension arrive ainsi dans le dispositif séparateur (filtre/centrifugeur) (8), permettant d'une part de récupérer un tourteau solide de sulfate d'ammonium impur contenant du AHMTB, qui est transporté par les moyens représentés par (O) jusqu'au récipient de dilution (10), d'où sort par la tuyauterie (P) une solution de sulfate d'ammonium avec une petite proportion de AHMTB, qui recircule jusqu'au récipient de neutralisation (3). D'autre part, à partir du dispositif séparateur (8), on retire par la tuyauterie (Q) du AHMTB liquide qui, après dilution avec de l'eau, pour adapter sa concentration dans le mélangeur (9) et éventuellement stabilisation avec de l' acide sulfurique, est recueilli comme produit principal du processus.

La phase aqueuse (Ph A), retirée du décanteur (4) par la tuyauterie (L), est concentrée dans l'évaporateur (5) dans lequel les références SV et EV ont les mêmes significations que dans l'évaporateur (7). Par la tuyauterie (R) on recueille l'effluent aqueux et par (S) une suspension aqueuse de sulfate d'ammonium impur contenant du AHMTB qui est séparée par filtration et/ou centrifugation en (6) pour retirer du sulfate d'ammonium sous-produit qui, après séchage, peut être utilisé comme fertilisant. Le liquide aqueux qui contient du sulfate d'ammonium qui n'a pas précipité et du AHMTB dissous, est renvoyé par la tuyauterie (T) jusqu' au récipient de neutralisation (3).

Dans le diagramme des dessins on a seulement représenté les éléments principaux, en ayant omis délibérément des dispositifs et accessoires usuels, tels que pompes, mesureurs de débit, moyens de chauffage, mesureurs de température, etc...

Comme on peut le voir, le procédé amélioré selon l'invention permet de ne pratiquement pas perdre de AHMTB dans des courants effluents, et on peut dire que la récupération est de 99 %.

Le procédé amélioré selon l'invention est extrêmement avantageux par rapport à la technique antérieure. En effet, le brevet US 2 745 745 décrit un procédé pour obtenir un sel d'un métal alcalino-terreux, le sel de calcium de l'acide AHMTB, au moyen de l'hydrolyse en deux étapes du HMTBN, en passant par l'amide correspondant, et en obtenant le sel par addition d'un hydroxyde ou d'un carbonate du métal alcalino-terreux. Cette méthode a le désavantage de nécessiter le changement des conditions d'hydrolyse du HMTBN, pour obtenir d'abord l'hydroxyamide correspondant et ensuite, sous d'autres conditions, pour obtenir le AHMTB. Nous avons trouvé que la solution acidifiée par notre procédé peut être hydrolysée en une seule étape avec des rendements pratiquement quantitatifs. Ceci est également valable pour le brevet US 2 983 053 qui concerne essentiellement la synthèse du AHMTB, en passant par l'hydroxyamide intermédiaire, pour obtenir le sel de calcium du AHMTB. Le brevet US 2 745 745 emploie une solution d'hydroxyde ou de carbonate de calcium, pour neutraliser l'acide sulfurique employé dans l'hydrolyse. Cette méthode présente l'inconvénient d'obliger à séparer le sulfate de calcium correspondant du reste de la solution en outre, ce sulfate précipite sous forme très fine, ce qui entraîne des pertes considérables de produit dans le tourteau obtenu. En plus, le sel alcalin correspondant du AHMTB est obtenu sous forme impure, ce qui oblige à chauffer le filtrat en ajoutant plus d'hydroxyde ou de carbonate, ce qui complique et rend plus cher le processus. Le brevet US 2 745 745 établit également que l'acide libre peut être directement obtenu à partir de la masse d'hydrolyse par extraction avec un solvant non-miscible à l'eau. Cependant, il semble que les rendements obtenus par le procédé extractif décrit dans ce brevet sont faibles ; il est donc douteux que ledit procédé ait une application pratique.

Le brevet US 3 175 000 améliore le rendement d'obtention du sel de calcium du AHMTB par les moyens suivants : addition de sulfate d'ammonium à la masse d'hydrolyse jusqu'à saturation ; extraction de la phase aqueuse avec un solvant polaire non-miscible dans l'eau, de préférence un éther ; mélange de l'extrait avec la phase organique ; évaporation du solvant; et addition d'un hydroxyde ou carbonate de calcium pour former le sel du AHMTB.

Le brevet GB 722 024 fournit également du sulfate de calcium dans le produit final avec les problèmes commentés auparavant.

Les demandes de brevets européens 0 142 448 et 0 143 100 décrivent des procédés d'extraction liquide-liquide avec un solvant non-miscible à l'eau pour séparer les sels formés durant l'hydrolyse du HMTBN, et discréditent le procédé d'évaporation à cause de la production d'une plus forte couleur et d'un plus grand contenu en oligomères, d'une consommation énergétique élevée et de difficultés durant la séparation des sels. Notre procédé obvie à tous les points critiqués dans lesdites demandes, car il n'y a pas augmentation de couleur ni d'oligomères si l'évaporation est faite soigneusement aux températures préconisées dans le procédé ; la consommation énergétique est moindre car il n'y a pas à évaporer d'azéotrope solvant-eau, vu que par notre procédé de décantation préalable à l'évaporation il n'y a qu'à évaporer l'eau contenue dans la phase organique. Le procédé décrit dans la présente invention n'utilise aucun type de procédé extracteur avec un solvant pour améliorer le rendement et/ou la qualité du produit final, comme cela se passe dans les références mentionnées

auparavant ; en fait, grâce à un procédé d'évaporation simple et peu coûteux, on aboutit à un produit bec des caractéristiques aussi bonnes ou même améliorées de couleur, d'odeur et de contenu en oligomères. En plus, le coût et la complexité de l'installation sont moindres, et le danger des solvants inflammables durant leur transport, leur emmagasinage et leur manipulation est totalement éliminé, vu que notre processus utilise à tout moment des solutions aqueuses.

Le brevet US 3 773 927 utilise de l'acide chlorhydrique pour hydrolyser le HMTBN en améliorant le procédé décrit dans le brevet US 2 745 745 qui emploie des solutions d'acide sulfurique comme agent d'hydrolyse. Etant donné que par hydrolyse avec de l'acide chlorhydrique on obtient une solution de AHMTB qui présente une seule phase, tout le chlorure d' ammonium produit par l'hydrolyse se trouve dans cette phase ; il s' ensuit que les procédés séparateurs subséquents sont très difficiles à réaliser avec des rendements élevés, vu les difficultés existantes dans le maniement de suspensions à viscosité élevée ; son application industrielle semble donc peu viable. En plus, nous avons pu vérifier que l'acide chlorhydrique ne semble pas approprié pour réaliser l'hydrolyse de l'hydroxynitrile, vu la grande quantité d'oligomères formés et vu la forte coloration du produit. Ceci oblige à le diluer et à le chauffer à 90°C en présence d'acide chlorhydrique, afin d' hydrolyser les oligomères formés.

Le brevet US 4 353 924 permet l'obtention d'une solution aqueuse de AHMTB. Cependant, la principale propriété cherchée pour la solution est qu'elle soit exempte de propriétés corrosives vis-à-vis des surfaces en acier. Bien qu'on envisage la neutralisation partielle du mélange d'hydrolyse, cette neutralisation n'est pas réalisée de manière impérative après l'hydrolyse ; elle peut être réalisée après déshydratation de la solution et avant la centrifugation, pour séparer le sel précipité de l'acide minéral (chlorure d'ammonium, sulfate d'ammonium, etc...) ou bien après la centrifugation comme étape finale. A partir du texte bref de ce brevet, il semble qu'on peut déduire un faible rendement dans la récupération du AHMTB. D'autre part, les conditions de neutralisation ne permettent pas la décantation des deux phases, et le problème que prétend résoudre ce brevet semble hors de propos puis qu'il est nécessaire de stabiliser les solutions aqueuses de AHMTB avec de petites quantités d'acide minéral libre, pour empêcher la formation d'oligomères.

Ainsi donc, le procédé de l'invention est avantageux par rapport aux procédés connus, tant du point de vue de la qualité du produit obtenu que de l'économie du processus ; en effet, on réalise la récupération du sulfate d'ammonium, sous-produit exclusivement de la phase aqueuse décantée dans laquelle la concentration du AHMTB est faible, de sorte que la récupération du AHMTB comme produit principal et du sulfate d'ammonium comme sous-produit est pratiquement quantitative.

L'invention est maintenant illustrée par les exemples suivants qui en aucun cas ne présentent un caractère limitatif.

## EXEMPLE 1

Dans un réacteur pourvu d'une chemise thermostatique, d'un agitateur, d'un thermomètre et d'un entonnoir d'addition, on a introduit 200 g de 3-méthylthio-propionaldéhyde, ensemble avec 0,4 ml de pyridine. En maintenant la température de 20°C par recirculation d'eau glycolée au travers de la chemise du réacteur, on a ajouté 300 g de solution aqueuse d'acide cyanhydrique à 20 %, en ayant soin que durant l'addition de cet acide ladite température ne soit pas dépassée. On a ensuite élevé la température à 45°C et l'a maintenue durant 15 minutes.

Dans le même réacteur et sous forte agitation, on a ajouté 175 g d'acide sulfurique à 98 % avec la plus grande vitesse d' addition possible, en veillant cependant à ne pas dépasser la température de 50°C. Après l'addition d'acide, on a maintenu à 50°C pendant encore 30 minutes, puis on a élevé rapidement la température à 90°C et on l'y a maintenue durant 120 minutes en réalisant en même temps un léger vide au réacteur. Durant ce temps on a éliminé 60 g de condensats. La réaction d'hydrolyse étant terminée, on a neutralisé l'acide sulfurique présent dans la masse réactionnelle avec de l'hydroxyde d'ammonium à 25 % de concentration et on a procédé à la séparation des deux phases formées par décantation. La température de neutralisation et de décantation ne dépassait pas à 70°C.

La phase aqueuse décantée a été amenée dans un rotateur de vapeur où, grâce à l'application de vide, on a évaporé 266 g d'eau et précipité 231 g de sulfate d'ammonium qui furent récupérés par filtration. 52 g d'eaux-mères provenant de la filtration du sulfate d'ammonium ont été conservés pour être recyclés à la réaction de l'exemple 2 suivant (recyclage 1).

L'eau présente dans la phase organique a été éliminée par évaporation sous vide, et la suspension de sulfate d'ammonium a été filtrée, fournissant ainsi 117 g de tourteau et 287 g de AHMTB. Le tourteau a été dissous dans 233 g d'eau et gardé pour être recyclé dans la réaction de l'exemple 2 suivant (recyclage 2). Par analyse du AHMTB obtenu, on a trouvé qu'il contenait 80 % en poids de AHMTB monomère, 12 % en poids d'oligomères, 1,1 % d'eau et 0,8 % d'ions sulfate. Le AHMTB obtenu a été dilué et stabilisé avec de l'eau acidulée avec de

l'acide sulfurique, et on a obtenu un produit contenant 88 % de AHMTB, 0,7 % de sulfate d'ammonium, 0,3 % d'acide sulfurique libre et présentant une coloration qui, mesurée en degrés APHA, était inférieure à 2000.

EXEMPLE 2

Dans un réacteur équipé d'une chemise thermostatique, d'un agitateur, d'un thermomètre et d'un entonnoir d'addition, on a introduit 125 g de 3-méthylthio-propionaldéhyde technique (98 %) et 0,3 ml de pyridine. En refroidissant le réacteur au moyen d'eau glycolée à -10°C, on a ajouté 188 g d'une solution aqueuse d'acide cyanhydrique à 20% dans un temps non inférieur à 20 minutes. Ce délai écoulé, on a élevé la température de la masse réactionnelle à 45°C en 10 minutes et on a maintenu la masse à ladite température durant 30 minutes de plus.

On a ensuite ajouté 131 g d'acide sulfurique à 98 % de concentration tout en maintenant le mélange sous violente agitation et refroidi à 50°C dans une boucle continue d'addition, où le mélange fut maintenu sous agitation durant 30 minutes de plus. La masse acidifiée a ensuite été chauffée à 90°C et maintenue à ladite température durant 180 minutes en éliminant, grâce à l'application de vide au réacteur, 32 g d'eau et de matières volatiles durant les 60 premières minutes. Le reste du temps on a maintenu le réacteur à l'ébullition au reflux à la température citée et sous un léger vide. La réaction d'hydrolyse terminée, on a ajouté les recyclages 1 et 2, puis on a neutralisé l'acide sulfurique par addition d'une solution aqueuse d'ammoniac à 25 % tout en refroidissant pour ne pas dépasser la température de 70°C et on a laissé décanter les deux phases en maintenant ladite température. Par décantation on a obtenu 414 g de phase aqueuse avec 192 g de sulfate d'ammonium ce qui correspond à 72 % du total, et 381 g de phase organique qui contient le reste du sulfate d'ammonium et 198 g de AHMTB, ce qui correspond à 94 % du total.

A partir de la phase aqueuse on a précipité le sulfate d' ammonium, par évaporation sous vide de 90 % de l'eau présente ; après séchage, on a obtenu 172 g de sulfate d' ammonium d'une richesse supérieure à 99,5 %, le rendement de récupération étant supérieur à 99 %. Les 52 g d'eaux-mères provenant de la filtration de la suspension ont été recyclés à la réaction suivante au niveau du réacteur de mélange et constituent le recyclage du conduit (T) du diagramme des dessins.

Par distillation sous vide on a éliminé l'eau de la phase organique jusqu'à siccité et on a obtenu 179 g de AHMTB avec une richesse de 98,5 % et 102 g de tourteau contenant 73 % de sulfate d'ammonium et 27 % de AHMTB. Ledit tourteau a été dissous avec une petite quantité d'eau et recyclé à la réaction suivante, au niveau du réacteur de mélange, pour sa récupération. Cette solution correspond au recyclage du conduit (P) du diagramme des dessins. On a ajusté le AHMTB avec de l'eau acidulée par de l'acide sulfurique pour donner 200 g d'une solution aqueuse stable ayant une concentration de 88 % en poids de AHMTB, une coloration de 1800 degrés APHA et une viscosité de 75 centistokes. Par analyse quantitative spécifique pour le AHMTB monomère dans le produit final, on a vérifié qu' il ne contenait pas moins de 80 % de ce monomère.

**Revendications**

1. Procédé pour préparer des solutions aqueuses de l'acide 2-hydroxy-4-méthylthio-butyrique (par la suite dénommé AHMTB) résultant de la réaction d'hydrolyse, en une seule étape, du 2-hydroxy-4-méthylthiobutyronitrile avec de l'acide sulfurique, caractérisé par la réalisation des opérations suivantes :

   a) neutraliser le mélange acide résultant de la réaction d'hydrolyse et les recyclages avec de l'hydroxyde d' ammonium ($NH_4OH$), de façon à former deux phases, une phase organique contenant une proportion principale de AHMTB et une proportion secondaire de sulfate d'ammonium, et une phase aqueuse contenant une proportion principale de sulfate d'ammonium et une proportion secondaire de AHMTB ;

   b) séparer les deux phases ;

   c) précipiter le sulfate d'ammonium de la phase aqueuse par évaporation de l'eau, à la pression atmosphérique ou réduite, puis séparer ledit sulfate d'ammonium par filtration et/ou centrifugation, et recycler jusqu' au réacteur de neutralisation le liquide résultant qui contient du sulfate d'ammonium non précipité et du AHMTB dissous ;

   d) concentrer la phase organique par évaporation de l'eau contenue dans cette phase pour obtenir une suspension de AHMTB qui contient une portion résiduelle de sulfate d' ammonium ;

   e) séparer par centrifugation et/ou décantation la suspension résultant de l'opération antérieure pour obtenir un AHMTB pratiquement exempt de sulfate d'ammonium et un tourteau de sulfate d'ammonium impur contenant du AHMTB ;

   f) dissoudre dans l'eau le tourteau de sulfate d'ammonium impur obtenu dans l'opération antérieure,

et recycler la solution résultante jusqu'au réacteur de neutralisation ; et
g) diluer avec de l'eau le AHMTB obtenu dans l'étape e) et éventuellement le stabiliser avec une petite quantité d'acide sulfurique.

2. Procédé selon la revendication 1, caractérisé en ce que l'hydroxyde d'ammonium employé dans l'opération (a) de neutralisation a une concentration de 20-35 % en poids et, éventuellement, est obtenu par injection de $NH_3$ gazeux dans le mélange de réaction d'hydrolyse.

3. Procédé selon la revendication 1 ou 2, caractérisé en ce que durant la neutralisation on contrôle la température du mélange pour qu'elle ne dépasse pas la valeur d'approximativement 70°C.

4. Procédé selon l'une des revendications 1 à 3, caractérisé en ce que dans l'opération (d) la proportion d'eau dans la phase organique est amenée jusqu'à une valeur de 0,5-1 % en poids par évaporation sous pression réduite.

5. Procédé selon l'une des revendications 1 à 4, caractérisé en ce que le sulfate d'ammonium produit dans l'opération (c), après séparation et séchage, est mis en valeur comme sous-produit pratiquement exempt de AHMTB.

## Patentansprüche

1. Verfahren zur Herstellung wäßriger Lösungen der 2-Hydroxy-4-methylthio-buttersäure (im folgenden als AHMTB bezeichnet), die der Hydrolysereaktion in einer einzigen Stufe von 2-Hydroxy-4-methylthio-butyronitril mit Schwefelsäure entstammt, gekennzeichnet durch die Durchführung der folgenden Maßnahmen:
(a) Neutralisation des der Hydrolysereaktion und den Recyclisierungen entstammenden, sauren Gemisches mit Ammoniumhydroxid ($NH_4OH$), derart, daß zwei Phasen gebildet werden, eine organische Phase, die einen Hauptanteil an AHMTB und einen Nebenanteil an Ammoniumsulfat enthält, und eine wäßrige Phase, die einen Hauptanteil an Ammoniumsulfat und einen Nebenanteil an AHMTB enthält;
(b) die Trennung der beiden Phasen;
(c) die Ausfällung von Ammoniumsulfat aus der wäßrigen Phase durch Verdampfen von Wasser bei atmosphärischem oder vermindertem Druck, hiernach die Abtrennung des Ammoniumsulfats durch Filtrieren und/oder Zentrifugation und das Recyclisieren zu dem Neutralisationsreaktor der resultierenden Flüssigkeit, die nicht-ausgefälltes Ammoniumsulfat und gelöstes AHMTB enthält;
(d) das Einengen der organischen Phase durch kontinuierliches Verdampfen des Wassers in dieser Phase, um eine Suspension von AHMTB zu erhalten, die einen verbliebenen Ammoniumsulfat-Anteil enthält;
(e) das Abtrennen durch Zentrifugieren und/oder Dekantieren der der vorhergehenden Maßnahme entstammenden Suspension, um ein AHMTB, das von Ammoniumsulfat praktisch frei ist,und einen unreinen AHMTB enthaltenden Ammoniumsulfatkuchen zu erhalten;
(f) die Auflösung des bei der vorhergehenden Maßnahme erhaltenen Kuchens von unreinem Ammoniumsulfat in Wasser und das Recyclisieren der resultierenden Lösung zu dem Neutralisationsreaktor; und
(g) das Verdünnen der in Stufe (e) erhaltenen AHMTB mit Wasser und gegebenenfalls die Stabilisierung mit einer geringen Menge Schwefelsäure.

2. Verfahren gemäß Anspruch 1, dadurch gekennzeichnet, daß das bei der Neutralisationsmaßnahme (a) verwendete Ammoniumhydroxid eine Konzentration von 20 bis 35 Gew.% besitzt und gegebenenfalls durch Einspritzung von gasförmigem $NH_3$ in das Hydrolysereaktionsgemisch erhalten wird.

3. Verfahren gemäß Anspruch 1 oder 2, dadurch gekennzeichnet, daß man während der Neutralisation die Temperatur des Gemisches kontrolliert, damit sie nicht den Wert von etwa 70°C überschreitet.

4. Verfahren gemäß einem der Ansprüche 1 bis 3, dadurch gekennzeichnet, daß bei der Stufe (d) der Anteil des Wassers in der organischen Phase auf einen Wert von 0,5 bis 1 Gew.% durch Verdampfen unter vermindertem Druck gebracht wird.

5. Verfahren gemäß einem der Ansprüche 1 bis 4, dadurch gekennzeichnet, daß das bei der Maßnahme (c)

gebildete Ammoniumsulfat nach Abtrennen und Trocknen als praktisch von AHMTB freies Nebenprodukt wiederverwertet wird.

## Claims

1. Process for preparing aqueous solutions of 2-hydroxy-4-methylthiobutyric acid (hereinafter designated HMTBA) resulting from the hydrolysis reaction, in a single stage, of 2-hydroxy-4-methylthiobutyronitrile with sulphuric acid, characterised in that the following operations are carried out:
   a) neutralising the acidic mixture resulting from the hydrolysis reaction and the recyclings with ammonium hydroxide ($NH_4OH$), so as to form two phases, an organic phase containing a predominant proportion of HMTBA and a secondary proportion of ammonium sulphate, and an aqueous phase containing a predominant proportion of ammonium sulphate and a secondary proportion of HMTBA;
   b) separating the two phases;
   c) precipitating the ammonium sulphate from the aqueous phase by evaporation of water, at atmospheric or reduced pressure, and then separating off the said ammonium sulphate by filtration and/or centrifuging, and recycling the resulting liquid, which contains unprecipitated ammonium sulphate and dissolved HMTBA, to the neutralisation reactor;
   d) concentrating the organic phase by evaporation of the water present in this phase in order to obtain an HMTBA suspension which contains a residual portion of ammonium sulphate;
   e) separating the suspension resulting from the previous operation, by centrifuging and/or settling, in order to obtain an HMTBA which is virtually free from ammonium sulphate and an impure ammonium sulphate cake containing HMTBA;
   f) dissolving in water the impure ammonium sulphate cake obtained in the previous operation and recycling the resulting solution to the neutralisation reactor; and
   g) diluting with water the HMTBA obtained in step e) and, it appropriate, stabilising it with a small quantity of sulphuric acid.

2. Process according to Claim 1, characterised in that the ammonium hydroxide used in the neutralisation operation (a) has a concentration of 20-35 % by weight and, optionally, is obtained by injecting gaseous $NH_3$ into the hydrolysis reaction mixture.

3. Process according to Claim 1 or 2, characterised in that the temperature of the mixture is controlled during the neutralisation to ensure that it does not exceed a value of approximately 70°C.

4. Process according to one of Claims 1 to 3, characterised in that, in operation (d), the proportion of water in the organic phase is adjusted to a value of 0.5-1 % by weight by evaporation under reduced pressure.

5. Process according to one of Claims 1 to 4, characterised in that the ammonium sulphate produced in operation (c) is recovered, after separating off and drying, as a by-product virtually free from HMTBA.

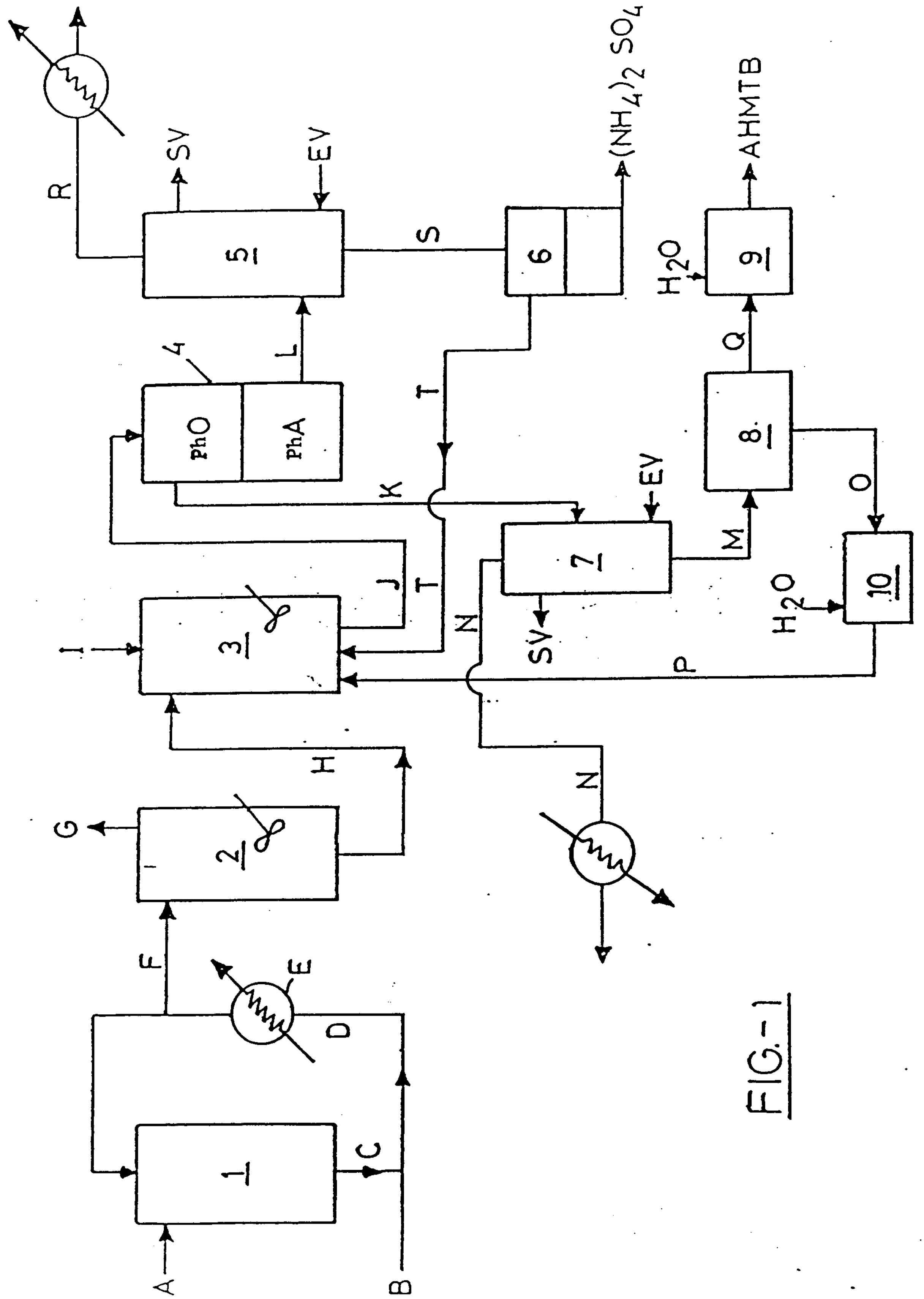
A
B
C
D
E
F
G
H
I
J
K
L
M
N
O
P
Q
R
S
T
SV
EV
PhO
PhA
(NH4)2 SO4
AHMTB
H2O
1
2
3
4
5
6
7
8
9
10

FIG.-1